# EUROPEAN PATENT SPECIFICATION

(11) **EP 1 352 970 B1**
(45) Date of publication and mention of the grant of the patent: **02.06.2010**
(21) Application number: 01270625.5
(22) Date of filing: 10.12.2001
(51) Int. Cl.: C12N 9/10, C12Q 1/68

(54) **METHOD OF ESTIMATING THE RISK OF EXPRESSION OF ADVERSE DRUG REACTION CAUSED BY THE ADMINISTRATION OF A COMPOUND, WHICH IS EITHER METABLOIZED PER SE BY UGT1A1 OR WHOSE INTERMEDIATE IS METABOLIZED BY THE ENZYME**
VERFAHREN ZUR ABSCHÄTZUNG DES RISIKOS DER EXPRESSION EINER DURCH DIE VERABREICHUNG EINER VERBINDUNG, DIE ENTWEDER PER SE DURCH UGT1A1 METABOLISIERT WIRD ODER DEREN ZWISCHENVERBINDUNG DURCH DAS ENZYM METABOLISIERT WIRD, HERVORGERUFENEN UNERWÜNSCHTEN ARZNEIMITTELWIRKUNG
PROCEDE D'ESTIMATION DU RISQUE DE L'EXPRESSION D'EFFETS SECONDAIRES CAUSES PAR L'ADMINISTRATION DE COMPOSE METABOLISE, SOIT AUTOMATIQUEMENT, SOIT COMME INTERMEDIAIRE METABOLIQUE, PAR L'ENZYME UGT1A1

(30) Priority: 12.12.2000 JP 2000376756
(43) Date of publication of application: 15.10.2003
(73) Proprietor: Nagoya Industrial Science Research Institute, Nagoya-shi, Aichi 460-0008 (JP); Sekisui Medical Co., Ltd., Tokyo 103-0027 (JP)
(72) Inventor: HASEGAWA, Yoshinori, Nagoya-shi, Aichi 464-0039 (JP); ANDO, Yu-uichi, Ogaki-shi, Gifu 503-0015 (JP); SHIMOKATA, Kaoru, Nagoya-shi, Aichi 463-0011 (JP)
(74) Representative: Müller Fottner Steinecke
(86) International application number: PCT/JP2001/010813
(87) International publication number: WO 2002/048400

(56) References cited:
- WO-A-97/32042
- WO-A-99/57322
- IYER L ET AL: "GENETIC PREDISPOSITION TO THE METABOLISM OF IRINOTECAN (CPT-11) ROLE OF URIDINE DIPHOSPHATE GLUCURONOSYLTRANSFERASE ISOFORM 1A1 IN THE GLUCURONIDATION OF ITS ACTIVE METABOLITE (SN-38) IN HUMAN LIVER MICROSOMES" JOURNAL OF CLINICAL INVESTIGATION, NEW YORK, NY, US, vol. 101, no. 4, 15 February 1998 (1998-02-15), pages 847-854, XP001120443 ISSN: 0021-9738
- INNOCENTI F ET AL: "PHARMACOGENETICS A TOOL FOR INDIVIDUALISING ANTINEOPLASTIC THERAPY" CLINICAL PHARMACOKINETICS, LEA & FEBIGER, PHILADELPHIA, PA, US, vol. 39, no. 5, November 2000 (2000-11), pages 315-325, XP009004869
- DATABASE OMIM [Online] NCBI; UDP-glycosyltransferase 1 family, polypeptide A1, retrieved from NCBI Database accession no. MIM 191740 XP002273617
- YAMAMOTO KAZUO ET AL: "Analysis of bilirubin uridine 5'-diphosphate (UDP)-glucuronosyltransferase gene mutations in seven patients with Crigler-Najjar syndrome type II" JOURNAL OF HUMAN GENETICS, vol. 43, no. 2, 1998, pages 111-114, XP002284547 ISSN: 1434-5161
- YAMAMOTO K ET AL: "Contribution of two missense mutations (G71R and Y486D) of the bilirubin UDP glycosyltransferase (UGT1A1) gene to phenotypes of Gilbert's syndrome and Crigler-Najjar syndrome type II" BIOCHIMICA ET BIOPHYSICA ACTA. MOLECULAR BASIS OF DISEASE, AMSTERDAM, NL, vol. 1406, no. 3, 28 April 1998 (1998-04-28), pages 267-273, XP004276748 ISSN: 0925-4439
- TUKEY R H ET AL: "HUMAN UDP-GLUCURONOSYLTRANSFERASES: METABOLISM, EXPRESSION, AND DISEASE" ANNUAL REVIEW OF PHARMACOLOGY AND TOXICOLOGY, ANNUAL REVIEW INC., PALO ALTO, CA, US, vol. 40, 2000, pages 581-616,2PAGES, XP009003008 ISSN: 0362-1642
- WASSERMAN E ET AL: "SEVERE CPT-11 TOXICITY IN PATIENTS WITH GILBERT'S SYNDROME: TWO CASE REPORTS" ANNALS OF ONCOLOGY, KLUWER, DORDRECHT, NL, vol. 8, no. 10, October 1997 (1997-10), pages 1049-1051, XP009026815 ISSN: 0923-7534
- IYER L. ET AL: 'Phenotype-genotype correlation of in vitro SN-38 (active metabolite of irinotecan) and bilirubin glucuronidation in human liver tissue with UGT1A1 promoter polymorphism' CLINICAL PHARMACOLOGY AND THERAPEUTICS vol. 65, no. 5, 1999, pages 576 - 582, XP002909099
- ANDO Y. ET AL: 'UGT1A1 genotypes and glucuronidation of SN-3 8, the active metabolite of irinotecan' ANNALS OF ONCOLOGY vol. 9, no. 8, 1998, pages 845 - 847, XP002909100
- AONO S. ET AL: 'Analysis of genes for bilirubin UDP-glucurono syltransferase in Gilbert's syndrome' THE LANCET vol. 345, 1995, pages 958 - 959, XP002909301
- ANDO Y. ET AL: 'Polymorphisms of UDP-glucuronosyltransferase gene and irinotecan toxicity: a pharmacogenetic analysis' CANCER RESEARCH vol. 60, 15 December 2000, pages 6921 - 6926, XP002909302
- MARUO Y. ET AL: 'Prolonged unconjugated hyperbilirubinemia associated with breast milk and mutations of the bilirubin uridine diphosphate-glucuronosyltransferase gene' PEDIATRICS vol. 106, no. 5, November 2000, page E59, XP002909303

### SEQUENCE LISTING

<110> Nagoya Industrial Science Research Institute
   Hasegawa, Yoshinori
   Ando, Yuichi
   Shimokata, Kaoru
<120> Method for predicting severe toxicity caused by dosing
   either a compound that is metabolized by UGT1A1 enzyme
   or a compound whose metabolite is metabolized by the
   enzyme.
<130> C0000101
<140>
   <141>
<150> JP P2000-376756
   <151> 2000-12-12
<160> 10
<170> Patent In Ver. 2.1
<210> 1
   <211> 33
   <212> DNA
   <213> Artificial Sequence
<220>
   <223> Description of Artificial Sequence:Primer for amplifying a 235-bp segment to analyze UGT1A1≠6
<400> 1
   ctagcacctg acgcctcgtt gtacatcaga gcc 33
<210> 2
   <211> 20
   <212> DNA
   <213> Artificial Sequence
<220>
   <223> Description of Artificial Sequence:Primer for amplifying a 235-bp segment to analyze UGT1A1≠6
<400> 2
   ccatgagctc cttgttgtgc 20
<210> 3
   <211> 19
   <212> DNA
   <213> Artificial Sequence
<220>
   <223> Description of Artificial Sequence:Primer for amplifying a 399-bp segment to analyze UGT1A1≠27
<400> 3
   agtacctgtc tctgcccac 19
<210> 4
   <211> 19
   <212> DNA
   <213> Artificial Sequence
<220>
   <223> Description of Artificial Sequence:Primer for amplifying a 399-bp segment to analyze UGT1A1≠27
<400> 4
   gtcccactcc aatacacac 19
<210> 5
   <211> 36
   <212> DNA
   <213> Artificial Sequence
<220>
   <223> Description of Artificial Sequence:Primer for amplifying a 285-bp to analyze UGT1A1≠29
<400> 5
   tcctccctat tttgcatctc aggtcacccg atggcc 36
<210> 6
   <211> 17
   <212> DNA
   <213> Artificial Sequence
<220>
   <223> Description of Artificial Sequence:Primer for amplifying a 285-bp to analyze UGT1A1≠29
<400> 6
   tgaatgccat gaccaaa 17
<210> 7
   <211> 21
   <212> DNA
   <213> Artificial Sequence
<220>
   <223> Description of Artificial Sequence:PCR Primer for analyzing TATA box region
<400> 7
   aagtgaactc cctgctacct t 21
<210> 8
   <211> 21
   <212> DNA
   <213> Artificial Sequence
<220>
   <223> Description of Artificial Sequence:PCR Primer for analyzing TATA box region
<400> 8
   ccactgggat caacagtatc t 21
<210> 9
   <211> 20
   <212> DNA
   <213> Artificial Sequence
<220>
   <223> Description of Artificial Sequence:PCR Primer for analyzing TATA box region
<400> 9
   gtcacgtgac acagtcaaac 20
<210> 10
   <211> 19
   <212> DNA
   <213> Artificial Sequence
<220>
   <223> Description of Artificial Sequence:PCR Primer for analyzing TATA box region
<400> 10
   tttgctcctg ccagaggtt 19

## Description

Method of estimating the risk of expression of adverse drug reaction caused by the administration of a compound, which is either metabolized per se by UGT1A1 enzyme or whose metabolic intermediate is metabolized by the enzyme

### TECHNICAL FIELD

The present invention relates to a method for estimating the risk of expression of adverse drug reaction of a drug by analyzing polymorphism of a gene encoding an enzyme involved in drug metabolism. Also, the present invention relates to a kit which is used for estimating the risk of expression of adverse drug reaction. Further, the present invention relates to a method for reducing the risk of expression of adverse drug reaction of a drug based on the results of the estimation of the risk of expression of adverse drug reaction.

More particularly, the present invention relates to a method for estimating the risk of expression of adverse drug reaction caused by the administration of a compound, which is either metabolized per se by UDP-GLUCURONOSYLTRANSFERASE (UGT) or whose metabolic intermediate is metabolized by UGT, by analyzing polymorphism of a gene encoding UGT and a kit for estimating the risk of expression of adverse drug reaction, as well as a method for reducing the risk of expression of adverse drug reaction.

### BACKGROUND ART

There are two types of UDP-glucuronosyltransferase (UGT) enzymes, UGT1 and UGT2, in humans and the UGT1 family consists of one gene along with multiple promoters and the first exons which are spliced to the mutual exon 2 (Ritter, J. K., Chen, F., Sheen, Y. Y., Tran, H. M., Kimura, S., Yeatman, M. T., and Owens, I. S., J. Biol. Chem., 267: 3257-3261, 1992). Thus, the substrate specificity of the enzyme depends on the first exon.

*UGT1A1* gene, which is one of the UGT1 family, is composed of a promoter and the first exon closest to exons 2 through 5.

UGT1A1 enzyme, which is primarily responsible for conjugating bilirubin, can glucuronidate drugs (e.g. ethinylestradiol), xenobiotic compounds (e.g. phenols, anthraquinones and flavones) and endogenous steroids (Senafi, S. B., Clarke, D.J., Burchell, B., Biochem. J., 303: 233-240, 1994). At present, not less than 30 genetic polymorphisms in a promoter region and exons have been known to decrease the enzyme activity and lead to constitutional unconjugated jaundice, Crigler-Najjar or Gilbert's syndrome (Mackenzie, P. I., et al., Pharmacogenetics, 7: 255-269, 1997). Recent in vitro analyses have revealed that UGT1A1 isoform would be responsible for the glucuronidation of SN-38 and that the genetic polymorphism would associate with the decreased activity of SN-38 glucuronidation as well as bilirubin glucuronidation (Iyer, L., et al., J. Clin. Invest., 101: 847-854, 1998, Iyer, L., et al., Clin. Pharmacol. Ther., 65: 576-582, 1999). Additionally, the present inventors have suggested an inter-individual difference in the pharmacokinetics of SN-38 and SN-38 glucuronide depending on *UGT1A1* genotype (Ando, Y., Saka, H., Asai, G., Sugiura, S., Shimokata, K., and Kamataki, T., Ann. Oncol., 9: 845-847,1998).

Tukey and Strassburg, Annu. Rev. Pharmacol. Toxicol. 40 (2000), 581-616 provide a review about the role of the UGTs in metabolism and different disease states in humans.

International application WO 99/57322 discloses genetic polymorphisms identified in the human UGT1 gene that alter UGT1-dependent drug metabolism.

International application WO 97/32040 describes a method for improving the efficacy of drug trials comprising the step of screening samples from potential participants for the genetic basis of the Gilbert's syndrome including studying UGT1 isoforms by determining the number of TA repeats in the promoter region.

Yamamoto et al., J. Hum. Genet. 43 (1998), 111-114 describe the analysis of bilirubin uridine 5'-diphosphate (UDP)-glucuronosyltransferase gene mutations in seven patients with Crigler-Najjar syndrome type II including the mutations Gly71Arg, Tyr486Asp, and Pro229Gln of the UGT1A1 gene.

One of the compounds, whose intermediate metabolites are metabolized (conjugated) by UGT1A1 enzyme, is irinotecan (CPT-11). Irinotecan is metabolized by carboxylesterase to form an active SN-38, which is further conjugated and detoxified by UGT1A1 to yield its β-glucuronide. The glucuronide is then excreted in the small intestine via bile, where bacterial glucuronidase resolves the glucuronide into the former SN-38 and glucuronic acid (Takasuna, K., Hagiwara, T., Hirohashi, M., Kato, M., Nomura, M., Nagai, E., Yokoi, T., and Kamataki, T., Cancer Res., 56: 3752-3757, 1996). Interindividual differences in pharmacokinetics of SN-38 are suggested to cause the variation in drug effect (Gupta, E., Lestingi, T. M., Mick, R., Ramirez, J., Vokes, E. E., and Ratain, M. J., Cancer Res., 54: 3723-3725, 1994, Kudoh, S., Fukuoka, M., Masuda, N., Yoshikawa, A., Kusunoki, Y., Matsui, K., Negoro, S., Takifuji, N., Nakagawa, K., Hirashima, T., Yana, T., and Takada, M., Jap. J. Cancer Res., 86: 406-413, 1995).

Irinotecan is a camptothecin analogue compound, and known for its strong antitumor activity through an inhibition of topoisomerase I. Although irinotecan is now widely used, especially for colorectal- and lung-cancer treatments, there are concerns about the dose limiting toxicity of irinotecan resulting in leukopenia and/or diarrhea (Negoro, S. et al., J. Natl. Cancer Inst., 83: 1164-1168, 1991, Akabayashi, A., Lancet, 350: 124, 1997, Pharmaceuticals and Cosmetics Division, Pharmaceutical Affairs Bureau, Ministry of Health and Welfare (ed), Summary Basis of Approval (SBA) No.1 (revised edition): irinotecan hydrochloride. Tokyo: Yakuji Nippo, Ltd., 1996). Adverse drug reaction of irinotecan is occasionally fatal (Rougier, P. et al., Lancet, 352: 1407-1412, 1998, Kudoh, S. et al., J. Clin. Oncol., 16: 1068-1674, 1998, Masuda, N. et al., Proc. Am. Soc. Clin. Oncol., 18: 459a, 1999, Negoro, S. et al., J. Natl. Cancer Inst., 83: 1164-1168, 1991), and there is actually a report of the deaths of 55 patients out of 1245 were attributed to the adverse drug reactions of irinotecan during period of its clinical trials (Akabayashi, A., Lancet, 350: 124, 1997, Pharmaceuticals and Cosmetics Division, Pharmaceutical Affairs Bureau, Ministry of Health and Welfare (ed), Summary Basis of Approval (SBA) No.1 (revised edition): irinotecan hydrochloride. Tokyo: Yakuji Nippo, Ltd., 1996).

Wasserman et al., Annals of Oncology 8 (1997), 1049-1051, present clinical evidence linking bilirubin glucuronidation status and irinotecan (CPT-11) related toxicity in patients with Gilbert's syndrome.

Innocenti et al., Clin. Pharmacokinet. 39 (2000), 315-325, review the association between irinotecan toxicity for cancer patients and the genetic differences in enzyme metabolism including polymorphisms of the UGT1A1 gene.

Ratain et al. proposed a method for reducing adverse drug reaction of irinotecan by using a compound which enhances the activity of UGT (USP No. 5786344).

### DISCLOSURE OF INVENTION

As described above, adverse drug reaction of the drug whose metabolism is associated with UGT1A1 enzyme is a serious problem. However, no effective means for estimating such adverse drug reaction is known. On the other hand, since a therapeutic index is limited in many cases in chemotherapy of cancer, it becomes very important to set a dose of a drug depending on individuals in order to reduce adverse drug reaction of a drug and enhance the effect thereof.

In view of such circumstances, an object of the present invention is to provide epoch-making means for reducing adverse drug reaction by the administration of a compound, such as irinotecan, which is either metabolized per se by UGT1A1 enzyme or whose metabolic intermediate is metabolized by the enzyme. That is, an object of the present invention is to provide a method for estimating the risk of expression of adverse drug reaction caused by the administration of a compound which is either metabolized per se by UGT1A1 enzyme or whose metabolic intermediate is metabolized by the enzyme; a kit utilizing the method; and a method for reducing the risk of expression of the adverse drug reaction of the compound.

In order to solve the above problems, the present inventors studied paying attention to polymorphism of a gene encoding UGT1A1 enzyme. More specifically, patients who had undergone the administration of irinotecan in cancer chemotherapy were investigated for correlation between polymorphism of *UGT1A1* gene and adverse drug reactions of irinotecan. In particular, polymorphisms of *UGT1A1* gene were studied in the promoter region, exon 1, exon 4 and exon 5. As a result, correlation was recognized between the adverse drug reaction of irinotecan and polymorphism due to the difference in the number of TA repeats in the promoter region or two kinds of polymorphisms due to one base substitution in exon 1 (211-positional base, 686-positional base). From such findings, it was suggested that there is correlation between these polymorphisms in *UGT1A1* gene, and an extent of adverse drug reaction by the administration of a compound which is either metabolized per se by UGT1A1 enzyme or whose metabolic intermediate is metabolized by the enzyme. Therefore, analysis of these polymorphisms of *UGT1A1* gene was considered to be effective means for estimating the risk of expression of adverse drug reaction caused by the administration of a compound, such as irinotecan, which is either metabolized per se by UGT1A1 enzyme or whose metabolic intermediate is metabolized by the enzyme. In particular, for the polymorphism in the promoter region and polymorphism due to substitution of a 686-positional base in exon 1, correlation was recognized between each of such polymorphisms individually and adverse drug reaction of irinotecan and, therefore, analysis of the two polymorphisms was considered independently to be effective means for estimating the risk of expression of adverse drug reaction caused by the administration of a compound which is either metabolized per se by UGT1A1 enzyme or whose metabolic intermediate is metabolized by the enzyme. The present invention relates to the embodiments as characterized in the claims and was made based on the above-mentioned findings and study results, with the following features:
1. A method for estimating the risk of expression of adverse drug reaction caused by the administration of a compound which is either metabolized per se by UGT1A1 enzyme or whose metabolic intermediate is metabolized by the enzyme, which comprises at least (a): a step of analyzing the number of TA repeats in the promoter region of a gene encoding UGT1A1 enzyme.
2. The method of 1, wherein the step of analyzing the number of TA repeats is a step of detecting any one of 5 through 8 as the number of TA repeats.
3. The method of 1, wherein the step of analyzing the number of TA repeats is a step of detecting either 6 or 7 as the number of TA repeats.
4. The method of any one of 1 to 3, which further comprises a step of amplifying a DNA containing the TA repeating region in the promoter region of a gene encoding UGT1A1 enzyme.
5. The method of any one of 1 to 4, which is a method for estimating the risk of expression of adverse drug reaction caused by the administration of a compound which is either metabolized per se by UGT1A1 enzyme or whose metabolic intermediate is metabolized by the enzyme, and which further comprises (b): a step of analyzing the base at nucleotide position 686 of a gene encoding UGT1A1 enzyme, and/or (c) a step of analyzing the base at nucleotide position 211 of a gene encoding UGT1A1 enzyme.
6. The method of 5, wherein the step of analyzing the base at nucleotide position 686 is a step of analyzing whether the base at nucleotide position 686 is cytosine or adenine.
7. The method of 5, wherein the step of analyzing the base at nucleotide position 211 is a step of analyzing whether the base at nucleotide position 211 is guanine or adenine.
8. The method of any one of 5 to 7, which further comprises a step of amplifying a DNA containing the base at nucleotide position 686 of a gene encoding UGT1A1 enzyme, and/or a DNA containing the base at nucleotide position 211 of a gene encoding UGT1A1 enzyme.
9. A method for estimating the risk of expression of adverse drug reaction caused by the administration of a compound which is either metabolized per se by UGT1A1 enzyme or whose metabolic intermediate is metabolized by the enzyme, which comprises at least a (b): a step of analyzing the base at nucleotide position 686 of a gene encoding UGT1A1 enzyme.
10. The method of 9, wherein the step of analyzing the base at nucleotide position 686 is a step of analyzing whether the base at nucleotide position 686 is cytosine or adenine.
11. The method either of 9 or 10, which further comprises a step of amplifying a DNA containing the base at nucleotide position 686 of a gene encoding UGT1A1 enzyme.
12. The method of any one of 1 to 11, wherein the compound is a camptothecin analogue compound.
13. The method of 12, wherein the camptothecin analogue compound is a camptothecin derivative.
14. The method of 13, wherein the camptothecin derivative is topotecan or irinotecan.
15. The method of 13, wherein the camptothecin derivative is irinotecan.
16. A method for setting a dose of the compound, which comprises a step of setting a dose of the compound based on the results of the method for estimating the risk of expression of adverse drug reaction of any one of 1 to 15.
17. A nucleic acid for analyzing the number of TA repeats in the promoter region of a gene encoding UGT1A1 enzyme, which hybridizes specifically with a DNA fragment derived from the region which contains bases of the TA repeating region of a gene encoding UGT1A1 enzyme and which can be amplified by PCR method using the primers of SEQ ID No. 7 and SEQ ID No. 8.
18. A nucleic acid for analyzing the number of TA repeats in the promoter region of a gene encoding UGT1A1 enzyme, which hybridizes specifically with a DNA fragment derived from the region which contains bases of the TA repeating region of a gene encoding UGT1A1 enzyme and which can be amplified by PCR method using the primers of SEQ ID No. 9 and SEQ ID No. 10.
19. A nucleic acid for analyzing the base at nucleotide position 211 of a gene encoding UGT1A1 enzyme, which hybridizes specifically with a DNA fragment derived from the region which contains the base at nucleotide position 211 of a gene encoding UGT1A1 enzyme and which can be amplified by PCR method using the primers of SEQ ID No. 1 and SEQ ID No. 2.
20. A nucleic acid for analyzing the base at nucleotide position 686 of a gene encoding UGT1A1 enzyme, which hybridizes specifically with a DNA fragment derived from the region which contains the base at nucleotide position 686 of a gene encoding UGT1A1 enzyme and which can be amplified by PCR method using the primers of SEQ ID No. 3 and SEQ ID No. 4.
21. A kit for estimating the risk of expression of adverse drug reaction caused by the administration of a compound which is either metabolized per se by UGT1A1 enzyme or whose metabolic intermediate is metabolized by the enzyme, which comprises a nucleic acid for analyzing the number of TA repeats in the promoter region of a gene encoding UGT1A1 enzyme.
22. The kit of 21, which further comprises a nucleic acid for analyzing the base at nucleotide position 686 of a gene encoding UGT1A1 enzyme, and/or a nucleic acid for analyzing the base at nucleotide position 211 of a gene encoding UGT1A1 enzyme.
23. A kit for estimating the risk of expression of adverse drug reaction caused by the administration of a compound which is either metabolized per se by UGT1A1 enzyme or whose metabolic intermediate is metabolized by the enzyme, which comprises a nucleic acid for analyzing the base at nucleotide position 686 of a gene encoding UGT1A1 enzyme.
24. The kit of any one of 21 to 23, wherein the compound is a camptothecin analogue compound.
25. The kit of 24, wherein the camptothecin analogue compound is a camptothecin derivative.
26. The kit of 25, wherein the camptothecin derivative is topotecan or irinotecan.
27. The kit of 25, wherein the camptothecin derivative is irinotecan.
28. A kit for estimating the risk of expression of adverse drug reaction of irinotecan in advance, which comprises at least either of (a): a nucleic acid for analyzing the number of TA repeats in the promoter region of a gene encoding UGT1A1 enzyme, or (b): a nucleic acid for analyzing the base at nucleotide position 686 of a gene encoding UGT1A1 enzyme.
29. The kit of 28, which is a kit for estimating the risk of expression of adverse drug reaction of irinotecan, and which further comprises a nucleic acid for analyzing the base at nucleotide position 211 of a gene encoding UGT1A1 enzyme.
30. A kit for estimating the risk of expression of adverse drug reaction of irinotecan, which comprises at least either (a): a nucleic acid for analyzing the number of TA repeats in the promoter region of a gene encoding UGT1A1 enzyme; or (b): a nucleic acid for analyzing the base at nucleotide position 686 of a gene encoding UGT1A1 enzyme, and which further comprises a reagent for amplifying a DNA containing a TA repeating region in the promoter region of a gene encoding UGT1A1 enzyme, or a DNA containing the base at nucleotide position 686 of a gene encoding UGT1A1 enzyme, which are to be analyzed.
31. The kit of 30, which is a kit for estimating the risk of expression of adverse drug reaction of irinotecan, which further comprises a nucleic acid for analyzing the base at nucleotide position 211 of a gene encoding UGT1A1 enzyme and a reagent for amplifying a DNA containing the base at nucleotide position 211 of a gene encoding UGT1A1 enzyme.

### BRIEF DESCRIPTION OF THE DRAWINGS

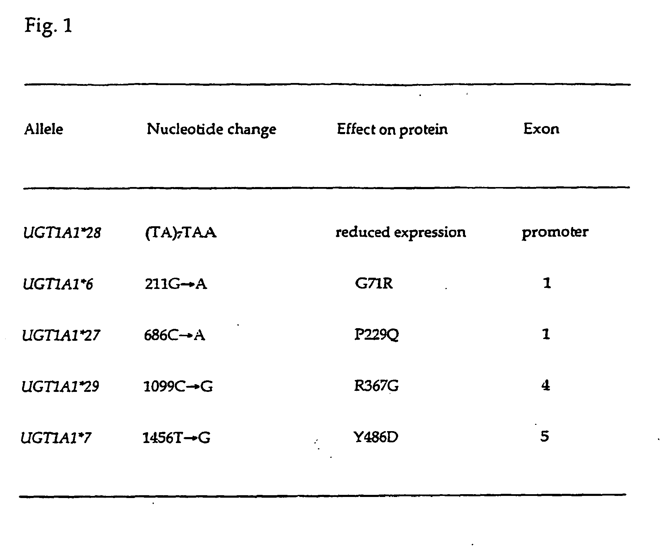

Fig. 1 is a table summarizing polymorphisms of *UGT1A1* gene which are analyzed in Examples. 211G—>A represents substitution of guanine by adenine at a position 211, 686C—>A represents substitution of cytosine by adenine at a position 686, 1099C—>G represents substitution of cytosine by guanine at a position 1099, and 1456T—>G represents substitution of thymine by guanine at a position 1456. In addition, G71R represents substitution of glycine by arginine at codon 71, P229Q represents substitution of proline by glutamine at codon 229, R367G represents substitution of arginine by glycine at codon 367, and Y486D represents substitution of tyrosine by aspartic acid.

Fig. 2 is a table summarizing clinical information of patients who are subjects in Examples. "a" is based on criteria of Japan Society of Clinical Oncology. In addition, "b" shows the results of a Chi-squared test, and C shows the results of a Mann-Whitney U test.

Fig. 3 is a table summarizing information of irinotecan chemotherapy of patients who are subjects in Examples. a: criteria of Japan Society of Clinical Oncology, b: Chi-squared test, c: Fisher's Exact test.

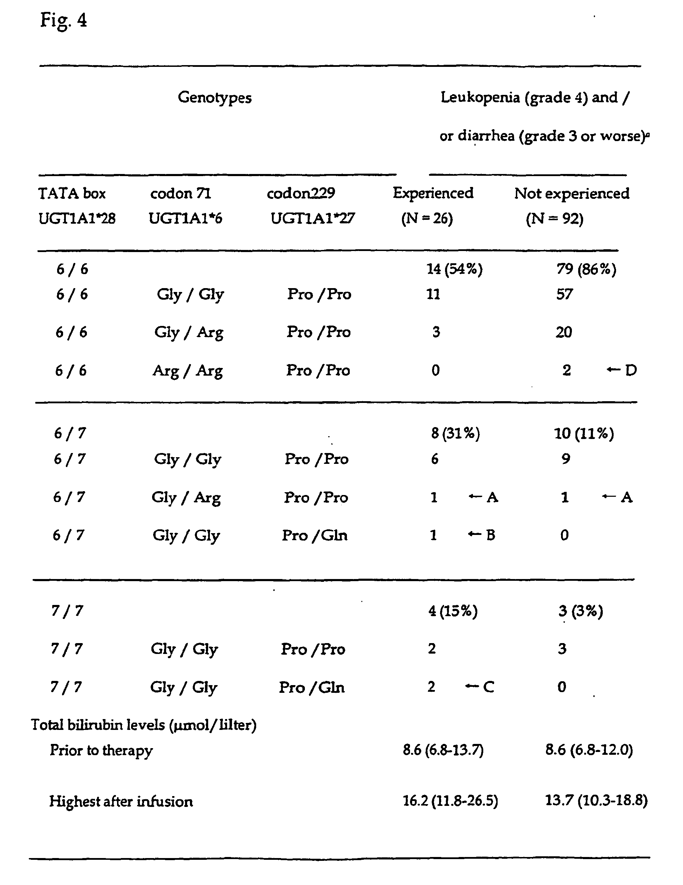

Fig. 4 is a table summarizing a distribution of genotype. 6/6 represents a homozygote of alleles in which the number of TA repeats is 6, 6/7 represents a heterozygote of an allele in which the number of TA repeats is 6 and an allele in which the number of TA repeats is 7, and 7/7 represents a homozygote of alleles in which the number of TA repeats is 7. Gly/Gly represents a homozygote of alleles in which codon 71 is glycine, Gly/Arg represents a heterozygote of an allele in which codon 71 is glycine and an allele in which codon 71 is arginine, and Arg/Arg represents a homozygote of alleles in which codon 71 is arginine. Pro/Pro represents a homozygote of alleles in which codon 229 is proline, and Pro/Gln represents a heterozygote of an allele in which codon 229 is proline and an allele in which codon 229 is glutamine. a: criteria of Japan Society of Clinical Oncology, b: average (inter-quartile range).

Fig. 5 is a table showing the results of statistically comparing (multiple logistic regression analysis) influence of *UGT1A1*28* and influence of other factors on severe toxicity. a. coefficient, b: regime of combination of irinotecan and other anti-cancer agent (except for platinum preparation).

### BEST MODE FOR CARRYING OUT THE INVENTION

The present invention relates to a method for estimating the risk of expression of adverse drug reaction caused by the administration of a compound which is either metabolized per se by UGT1A1 enzyme or whose metabolic intermediate is metabolized by the enzyme, which comprises (a): a step of analyzing the number of TA repeats in the promoter region of a gene encoding UGT1A1 enzyme; and (b): a step of analyzing the base at nucleotide position 686 of a gene encoding UGT1A1 enzyme.

UGT1A1 enzyme is a molecule species of UDP (uridine diphosphate)-glucronosyltransferase (UGT). UGT is a generic name of enzymes which catalyze glucronic acid conjugation (glucronide conjugation) of endogenous substances such as bilirubin and steroid, drugs having a particular structure and the like in a living body, and is involved in detoxication of many drugs. UGT1A1 is known to be deeply involved in metabolism of irinotecan as described above.

A gene encoding UGT1A1 enzyme (hereinafter referred to as *"UGT1A1* gene", Gen Bank Accession No.: AF297093) has a promoter region, exon 1, and exon 2 to exon 5 which are arranged subsequent to exon 1. It is known that a plurality of polymorphisms are present in the promoter region, and exon 1 to exon 5.

Polymorphism in the promoter region is due to a difference in the number of repeats of a pair of bases (TA), (TA repeats), and there are polymorphisms which are called (TA)₅, (TA)₆, (TA)₇ and (TA)₈ (which represent the number of TA repeats present: 5, 6, 7, 8, respectively) (Monaghan, G. et aL, Lancet, 347:578-581, 1996, Bosma, P.J. et al., N. Engl. J. Med., 333:1171-1175, 1995, Lampe JW et al., Pharmacogenetics, 9, 341-349, 1999).

"Analyzing the number of TA repeats" in the present invention means detecting the number of TA repeats in the promoter region of a test gene: which includes detecting any one of 5 through 8 as the number of TA repeats; detecting either 6 or 7 as the number of TA repeats.

In addition, a method of the present invention can be constituted by inclusion of the above-mentioned steps (a) and (b), and the step (c): a step of analyzing the base at nucleotide position 211 of a gene encoding UGT1A1 enzyme. Herein, the base at nucleotide position 686 is the 686th base when counting from a transcription initiation site in the downstream direction in *UGT1A1* gene; and the base at nucleotide position 211 is the 211th base in the same way.

The step (b) is a step of analyzing the base at nucleotide position 686 of a gene encoding UGT1A1 enzyme. It is known that there is polymorphism at nucleotide position 686 with two kinds of bases cytosine (C) or adenine (A) (Aono, S. et al., Lancet, 345:958-959, 1995). Therefore, "analyzing the base at nucleotide position 686 of a gene encoding UGT1A1 enzyme" means, in particular, to determine which, cytosine or adenine, is the base at nucleotide position 686.

The step (c) is a step of analyzing the base at nucleotide position 211 of a gene encoding UGT1A1 enzyme. It is known that there is polymorphism at the base at nucleotide position 211 with two kinds of bases, guanine (G) or adenine (A) (Aono, S. et al., Lancet, 345:958-959, 1995). Therefore, "analyzing the base at nucleotide position 211 of a gene encoding UGT1A1 enzyme" means, in particular, to determine which, guanine or adenine, is the base at nucleotide position 211.

In the determination of the number of TA repeats in the promoter region, of the base at nucleotide position 686, and/or of the base at nucleotide position 211 as described above, both alleles can be the target for analysis.

Method of analyzing the number of TA repeats, and method of analyzing the base at nucleotide position 686 or 211 are not particularly limited, but the known analyzing methods such as PCR-RFLP (polymerase chain reaction-restriction fragment length polymorphism) method utilizing PCR method, PCR-SSCP (single strand conformation polymorphism), (Orita, M. et al., Proc. Natl. Acad. Sci., U.S.A. 86, 2766-2770 (1989) etc.), PCR-SSO (specific sequence oligonucleotide) method, ASO (allele specific oligonucleotide) hybridization method in which PCR-SSO method and a dot hybridization method are combined (Saiki, Nature, 324, 163-166 (1986) etc.), TaqMan-PCR method (Livak, KJ, Genet Anal, 14, 143 (1999), Morris, T. et al., J. Clin., Microbiol., 34, 2933 (1996)), Invader method (Lyamichev V et al., Nat Biotechnol, 17, 292 (1999)), MALDI-TOF/MS (matrix) method using a primer elongation method (Haff LA, Smirnov IP, Genome Res 7, 378 (1997)), RCA (Rolling circle amplification) method (Lizardi PM et al., Nat Genet 19, 225 (1998)), a method using DNA chips or microarrays (Wang DG et al., Science 280, 1077 (1998) etc.), a primer elongation method, a southern blot hybridization method, a dot hybridization method (Southern, E., J. Mol. Biol. 98, 503-517 (1975)) and the like can be used. Further, analysis may be performed by directly sequencing the relevant parts of the sequences. These methods may be used by arbitrarily combining with one another. In addition, when at least two steps of (a) to (c) are performed to estimate the risk of expression of adverse drug reaction, not only the same analyzing method for all steps but also the different methods arbitrarily selected for each step can of course be used.

When the amount of a test DNA is small, it is preferable to perform analysis by PCR-RFLP method utilizing PCR method and the like, from the viewpoint of detection sensitivity or precision. Alternatively, after a test DNA is amplified in advance by a PCR method or a gene amplifying method according to a PCR method, any of the above-mentioned analyzing methods can be applied. On the other hand, when a number of test DNAs are analyzed, in particular, it is preferable to use a TaqMan-PCR method, an Invader method, a MALDI-TOF/MS (matrix) method using a primer elongation method, a RCA (rolling circle amplifying method), or a method utilizing a DNA tip or a microarray.

*UGT1A1* gene can be obtained from blood, skin cell, mucosal cell, hair and the like of a subject by the extraction method or purification method within the public domain. In addition, any of the genes containing the base part which is analyzed in the present invention, whether its DNA is full-length or partial, can be used as *UGT1A1* gene in the present invention. In other word, in a step of analyzing the number of (TA) repeats in the promoter region, a DNA fragment having an arbitrary length can be used as far as it contains the repeats part. In addition, in a step of analyzing the base at nucleotide position 686, a DNA fragment of an arbitrary length can be used as far as it contains the relevant base part. Similarly, in a step of analyzing the base at nucleotide position 211, a DNA fragment having an arbitrary length can be used as far as it contain the relevant base part.

In addition, analysis in each step may be performed using the mRNA which is the transcription product of *UGT1A1* gene. In this case, for example, the mRNA of *UGT1A1* gene is extracted and purified from blood or the like of a subject and, thereafter, the cDNA is prepared by reverse transcription. And, by analyzing the base sequence of the cDNA, sequences of the parts relating to polymorphism of a genome DNA is estimated in advance.

Further, two polymorphisms in exon 1 may be determined by using an expression product of *UGT1A1* gene. That is, by analyzing an expression product (amino acid) of a polymorphism part of exon 1, its genotype can be determined. In this case, as far as the polymorphism part of exon 1 contains the corresponding amino acids, even a partial peptide can be measured. Specifically, since polymorphism at a position 211 of exon 1 can change codon 71 (generating glycine or arginine), a peptide at least containing an amino acid corresponding to codon 71 may be used as a subject to be measured. Similarly, since polymorphism at a position 686 of exon 1 changes codon 229 (generating proline or glutamine), a peptide at least containing an amino acid corresponding to codon 229 can be used as a subject to be measured. When a peptide or a protein containing both of an amino acid corresponding to codon 71 and an amino acid corresponding to codon 229 is used, it is possible to analyze two polymorphisms simultaneously.

As a method of analyzing an amino acid corresponding to a polymorphism part using a peptide or a protein, the well known amino acid sequence analyzing method (a method utilizing the Edman method) can be used. In addition, it is estimated that conformation of an expression product of *UGT1A1* gene will change due to the change of an amino acid, the kind of amino acid may be analyzed by immunological methods. Examples of the immunological method include ELISA method (enzyme-linked imunosorbent assay), radioimmunoassay, immunoprecipitation method, immunodiffusion method and the like.

The "compound which is either metabolized per se by UGT1A1 enzyme or whose metabolic intermediate is metabolized by the enzyme" refers to a compound which is directly metabolized in vivo by UGT1A1 enzyme when administered to the living body, or a compound in which said compound is once metabolized by enzymes or the like, and its resulting metabolite (intermediate metabolite) is metabolized by UGT1A1 enzyme. The compound is not particularly limited as far as it is a compound having such nature; for example, a camptothecin analogue compound corresponds to the compound. Any compound is applicable without limit as far as it is a camptothecin analogue compound having the above-mentioned nature; for example, the known camptothecin derivative such as topotecan, irinotecan (CPT-11) and the like. Further, under the conditions that the above-mentioned nature is maintained, the compound may be a compound in which one or a few substituent(s) is (are) substituted with other atom(s) or atomic group(s) in the known camptothecin analogue compounds, and camptothecin derivatives (topotecan, irinotecan etc.) within the public domain.

A suitable example of the compound which is either metabolized per se by UGT1A1 enzyme or whose metabolic intermediate is metabolized by the enzyme is irinotecan.

A risk of the expression of adverse drug reaction refers to the risk that adverse drug reaction is caused due to the administration of the compound in the present invention. Herein, the adverse drug reaction refers to the action or effect other than the effect (drug efficacy) expected when the compound in the present invention is administered, and include not only adverse influence on a living body but also reduction in the effect inherent in the compound. Therefore, in the method of the present invention, the risk of reducing the inherent effect in the administration of the compound is also included in the risk of expression of adverse drug reaction.

An example of adverse drug reactions when the compound of the present invention is irinotecan is leucopenia and diarrhea. These symptoms have occasionally lethal adverse influence on the patient who has been dosed with irinotecan.

The results of estimating the risk of expression of adverse drug reaction can be utilized for setting a dose of the compound. Then, another aspect of the present invention is a method of setting a dose of the compound, which comprises a step of setting a dose of the compound based on the results of the above-mentioned method of estimating the risk of expression of adverse drug reaction. According to such method of setting a dose, it is possible to set a proper dose for every subject (patient) dosed, by comparing the degree of adverse drug reaction caused by the administration of the compound and the degree of the effect which is originally expected by the administration of the compound. Therefore, it becomes possible to perform effective therapies with the compound while suppressing the occurrence of adverse drug reactions. In other words, a method for reducing adverse drug reaction of the compound is provided.

Another aspect of the present invention provides a nucleic acid for analyzing the number of TA repeats in the promoter region of a gene encoding UGT1A1 enzyme (hereinafter referred to as "TA repeating number analyzing nucleic acid"), a nucleic acid for analyzing the base at nucleotide position 686 of a gene encoding UGT1A1 enzyme (hereinafter referred to as "686-position analyzing nucleic acid"), and a nucleic acid for analyzing the base at nucleotide position 211 of a gene encoding UGT1A1 enzyme (hereinafter referred to as "211-position analyzing nucleic acid").

Examples of the TA repeating number analyzing nucleic acid include a nucleic acid that hybridizes specifically with a DNA fragment derived from the region which contains the bases of TA repeating region of a gene encoding UGT1A1 enzyme and which can be amplified by PCR method using either of the following two primer sets: a set of SEQ ID No. 7 and SEQ ID No. 8, or a set of SEQ ID No. 9 and SEQ ID No. 10.
Forward primer (SEQ ID No. 7)
   5'-AAGTGAACTCCCTGCTACCTT-3'
Reverse primer (SEQ ID No. 8)
   5'-CCACTGGGATCAACAGTATCT-3'
Forward primer (SEQ ID No. 9)
   5'-GTCACGTGACACAGTCAAAC-3'
Reverse primer (SEQ ID No. 10)
   5'-TTTGCTCCTGCCAGAGGTT-3'

Examples of the 686-position analyzing nucleic acid include a nucleic acid that hybridizes specifically with a DNA fragment derived from the region which contains the base at nucleotide position 686 of a gene encoding UGT1A1 enzyme and which can be amplified by PCR method using the following primer set: a set of SEQ ID No. 3 and SEQ ID No. 4.
Forward primer (SEQ ID No. 3)
   5'-AGTACCTGTCTCTGCCCAC-3'
Reverse primer (SEQ ID No. 4)
   5'-GTCCCACTCCAATACACAC-3'

Examples of the 211-position analyzing nucleic acid include a nucleic acid that hybridizes specifically with a DNA fragment derived from the region which contains the base at nucleotide position 211 of a gene encoding UGT1A1 enzyme and which can be amplified by PCR method using the following primer set: a set of SEQ ID No.1 and SEQ ID No. 2.
Forward primer (SEQ ID No. 1)
   5'-CTAGCACCTGACGCCTCGTTGTACATCAGAGCC-3'
Reverse primer (position 393 to 412) (SEQ ID No. 2)
   5'-CCATGAGGTCCTTGTTGTGC-3'

Another aspect of the present invention provides a kit for use in a method as characterized above for estimating the risk of expression of adverse drug reaction caused by the administration of a compound which is either metabolized per se by UGT1A1 enzyme or whose metabolic intermediate is metabolized by the enzyme, which comprises a nucleic acid for analyzing the number of TA repeats in the promoter region of a gene encoding UGT1A1 enzyme (TA repeating number analyzing nucleic acid).

On the other hand, by inclusion of a nucleic acid for analyzing the base at nucleotide position 686 of a gene encoding UGT1A1 enzyme (686-position analyzing nucleic acid), constituted can be a kit for estimating the risk of expression of adverse drug reaction caused by the administration of a compound which is either metabolized per se by UGT1A1 enzyme or whose metabolic intermediate is metabolized by the enzyme.

Alternatively, a kit of the present invention can be constituted by inclusion of the 686-position analyzing nucleic acid in addition to the TA repeats number analyzing nucleic acid. Furthermore, by further inclusion of a nucleic acid for analyzing the base at nucleotide position 211 of a gene encoding UGT1A1 enzyme (211-position analyzing nucleic acid) in addition to the TA repeats number analyzing nucleic acid, a kit of the present invention may be constituted. In addition, the above-mentioned respective kits may be constituted by a reagent or 2 or more reagents combined depending on a method of using each kit. For example, a kit may be constituted by combining a reagent for amplifying a DNA containing a TA repeats number region of a gene encoding UGT1A1 enzyme, a reagent for amplifying a DNA containing the 686-positional base region of a gene encoding UGT1A1 enzyme, and/or a reagent for amplifying a DNA containing the 211-positional base region of a gene encoding UGT1A1 enzyme.

The above nucleic acids are the nucleic acids used in the respective analyzing methods, PCR-RFLP (restriction fragment length polymorphism) method, a PCR-SSCP (single strand conformation polymorphism) method (Orita, M. et al., Proc. Natl. Acad. Sci., U.S.A., 86, 2766-2770(1989) etc.) etc) as previously mentioned, which is utilized in each kit: primers and probes. Examples of the primers include primers which can specifically amplify the region containing a polymorphism site which is to be analyzed (promoter region, 686-positional base, 211-positional base). In addition, when a kit for conducting PCR-RFLP method is constituted, for example, primers are used which are designed so that a particular restriction site is formed in a polymorphism part when a particular polymorphism is possessed; such that a genotype is discriminated when a PCR amplification product is subjected to the restriction enzyme treatment. When a kit for conducting TaqMan-PCR method, Invader method or the like is constituted, examples of the nucleic acid include a primer and/or a probe which are used in each method.

As a probe or a primer, a DNA fragment or a RNA fragment is appropriately used depending on the analyzing method. The base length of a probe or a primer may be such a length that each function is exerted, and an example of the base length of a primer is around 15 to 30bp, and preferably around 20 to 25bp.

As described above, in order to easily implement the method of the present invention, it is preferable to use a genotype detecting kit suitable for this. Such kit can be easily designed based on the above explanation, and will be further explained by way of an example of Invader method which can implement an assay using a genome DNA fundamentally without amplifying a test DNA by PCR method or the like.

When a kit in accordance with Invader method is constituted, two kinds of non-fluorescently-labeled oligonucleotides (1) and (2), one kind of fluorescently-labeled oligonucleotide (3) and the enzyme having the specific endonuclease activity which recognizes and cuts a structure of a DNA (4) are used. Two kinds of non-fluorescently-labeled oligonucleotides are referred to as "allele probe (or signal probe or reporter probe)" and "invader probe" respectively, the fluorescently-labeled oligonucleotide is referred to as "FRET probe" and the enzyme having the specific endonuclease activity which recognizes and cuts the structure of a DNA is referred to as "clevase".
(1) The allele probe is designed so that it has the base sequence complementary to the 5' side from a polymorphism site (hereinafter referred to as "polymorphism site") to be analyzed in a gene encoding UGT1A1 enzyme (hereinafter also abbreviated as *UGT1A1* gene") as a template, and has an arbitrary base sequence (called as "flap") which can not complementarily bind to one base 3' side from the polymorphism site. More specifically, when looking at the allele probe itself, it is constituted in the following order starting from its 5'-terminal: the flap part, and the sequence part which is complementary to the sequence of the template in its 5'-side from the polymorphism site. As a sequence of the flap, used is a sequence which can not complementarily bind to *UGT1A1* gene sequence, the allele probe or the invader probe, and any DNAs other than the *UGT1A1* gene in a sample.
(2) The invader probe is designed so that it complementarily binds to the sequence from a polymorphism site to 3' side in its template *UGT1A1* gene, and a sequence corresponding to the polymorphism site may be an arbitrary base (N). More specifically, when looking at the invader probe itself, it is constituted in the following order starting from its 5'-terminal: the sequence part which is complementary to the sequence of the template in its 3'-side from the polymorphism site, and a N at the 3'-terminal.
   The (1) and (2) constituted in this manner correspond to the "TA repeating number analyzing nucleic acid", the "686-position analyzing nucleic acid" or the "211-position analyzing nucleic acid" of the present invention. When two kinds of probes, (1) and (2), and the *UGT1A1* gene are complementarily bound, an invader probe can invade the polymorphism sites in between, its single base (N) creating a structure with a base-pair overlap.
(3) A FRET (fluorescence resonance energy transfer) probe can be constituted by a sequence having no relationship with the *UGT1A1* gene, and the sequence of the FRET probe may be common not depending on the polymorphism sites which is intended to be detected. The FRET probe has the sequence to which the probe itself can complementarily bind on its own 5' side, and has the sequence which is complementary to a flap on the 3' side. In addition, the 5'-terminal of the FRET probe is labeled with a fluorescent pigment, and a quencher is bound to its upstream site.
(4) Clevase is an enzyme having the specific endonuclease activity which is classified as a structure-specific flap endonuclease (FEN); and recognizes and cuts the structure of a DNA; detects the part where three bases are arrayed, each belonging to a template DNA, an invader probe and an allele probe, and where a 5'-terminal of allele probe is flap-like; and cuts the flap part.

When the above-mentioned three kinds of probes (1) to (3) and cleavase (4) are used, the following two-stage reaction is schematically generated.

First, when the allele probe (1) and the *UGT1A1* gene are complementarily bound, the 3'-terminal (N) of the invader probe (2) invades their polymorphism site. The cleavase (4) recognizes a structure of the polymorphism site in which these three bases are arrayed, cuts a flap part of the allele probe (1), and the flap part is released. Then, the flap part released from the allele probe (1) complementarily binds to a FREP probe (3) because the flap part has the sequence which is complementary to the FREP probe (3). Upon this, the polymorphism site present at the 3'-terminal of the flap part invades into a complementarily self-binding site of the FRET probe (3). The cleavase (4) in turn recognizes this structure, and cuts the site which is bound with a fluorescent pigment. Thereby, the fluorescent pigment is separated from the quencher and, therefore, emits the fluorescent light. This fluorescent intensity is measured to detect and analyze the polymorphism.

(1) to (4) may be used, for example, by combining (1) and (2), or (3) and (4) as a composition of two kinds of reagents, or (3) and (4) may be dried in advance to be encapsulated into a microtiter plate. Thereby, the number of steps for assay can be reduced. Moreover, magnesium, a buffer and the like may be appropriately incorporated into a reagent composition containing (1) and (2), to optimize a reaction. Further, in addition to (1) to (4), a mineral oil for preventing a sample from evaporating during measurement may be combined to obtain a kit.

In addition, if two kinds of probes are prepared for the allele probe (1), whether the *UGT1A1* gene is homozygote or heterozygote can be distinguished. Since these are diagramed and described in Post-Sequence Genome Science "Strategy for SNP Gene Polymorphism", Yozo Onishi, 94-135, Nakayamashoten, 2000; Treble, M., et al., Genetic Medicine, 4:68-72, 2000, Outline; and WO97/27214 and WO98/42873 which are International Publications, it is possible to perform optimum design by referring to these publications.

Since the risk of expression of adverse drug reaction caused by the administration of a compound which is either metabolized per se by UGT1A1 enzyme or whose metabolic intermediate is metabolized by the enzyme can be estimated in advance the kit of the present invention, a proper dose of the compound can be set for every administration subject based on the estimation results. In other words, the kit of the present invention can be used for setting a dose of a compound which is either metabolized per se by UGT1A1 enzyme or whose metabolic intermediate is metabolized by the enzyme.

By introducing a *UGT1A1* gene having a genetic polymorphism which is determined to have less risk of expression of adverse drug reaction (in other words, having six TA repeats in the promoter region, guanine (G) at position 211 of exon 1, and/or cytosine at position 686 of exon 1), or a DNA fragment containing at least one of those polymorphism sites into a cell of a patient receiving administration of a compound, in particular, into a cell at a site where UTG1A1 enzyme acts on metabolism and detoxication of the compound; a risk of expression of the adverse drug reaction of the compound can be reduced. Introduction of the genes can be performed before administration, during administration, or after administration of the compound. Introduction of the genes can be performed, for example, by the methods such as a method using a plasmid or a virus vector for introducing a gene, electroporation (Potter, H. et al., Proc. Natl. Acad. Sci. U.S.A. 81, 7161-7165 (1984)), lipofection (Felgner, P. L. et al., Proc. Natl. Acad. Sei. U.S.A. 84, 7413-7417 (1984)), microinjection (Graessmann, M. & Graessmann, A. Proc. Natl. Acad. Sci. U.S.A. 73, 366-370 (1976)) and the like.

The present invention will be explained in more detail by means of Example. In this Example, the correlation between the adverse drug reaction due to irinotecan administration and polymorphisms of *UGT1A1* genes was statistically analyzed.

### [Patients and Clinical Information]

The subjects were Japanese patients who had received irinotecan administration in their chemotherapies from July 1994 to June 1999. For confirming the security of the patients, each patient was primarily ensured to have an adequate bone marrow function before the use of irinotecan: a leukocyte count of 3x10⁹/liter or more, and a platelet count of 100x10⁹/liter or more. In addition, the patients who had evidence of watery diarrhea, paralytic ileus, pulmonary interstitial pneumonia or fibrosis, massive ascites or pleural effusion, apparent jaundice, or anamnesis of hypersensitivity to irinotecan were excluded from the irinotecan use. As a result, 118 patients were used as a subject of this Example.

For confirming the adaptability to irinotecan, the complete blood count, platelet count and serum chemistry were assessed at least once a week. And bilirubin levels were always measured.

We retrospectively reviewed the clinical records including patients' characteristics (e.g. age, gender and the like), the dosage and schedule of irinotecan administration, the record of use of other drugs or radiotherapy, and observed adverse drug reaction caused by irinotecan (infusion). We counted the number of days when the patients received granulocyte colony stimulating factors (G-CSFs) or loperamide hydrochloride, the latter of which is commonly prescribed for irinotecan-induced diarrhea in Japan. Prophylactic administration of G-CSF prevented the apparent onset of neutropenia. Since the dose limiting toxicity of irinotecan is known to result in leukopenia and diarrhea, we defined as "severe toxicity" as leukopenia of grade 4 (≤0.9x10⁹/liter) and the diarrhea of grade 3 or worse (grade 3 is for the watery diarrhea for 5 days or more; grade 4, the diarrhea with hemorrhagic or dehydration as classified in accordance with the Japan Society for Cancer Therapy criteria). No other adverse drug reactions were included in this example because they would be influenced by miscellaneous patients' backgrounds. The Serum total bilirubin levels were recorded with the scores just prior to irinotecan administration along with the highest of those after initiation of the therapy.

### [Genotyping]

Blood was sampled from each patient (118 samples) and their genotypes were analyzed after irinotecan administration in each patient. First, genomic DNA was prepared from the whole blood (100-200 µl) using QIAamp Blood Kit (QIAGEN GmbH, Hilden, Germany). Then the assay was performed according to the accompanying manual.

We analyzed the following variant sequences for each genomic DNA (see Fig. 1): a two-extra-nucleotide (TA) insertion within the TATA box resulting in the sequence (TA)₇TAA at positions -39 to -53, whose type is referred to as *UGT1A1***2*8; (Monaghan, G., Ryan, M., Seddon, R., Hume, R., and Burchell, B., Lancet, 347: 578-581, 1996, Bosma, P. J. et al., N. Engl. J. Med., 333: 1171-1175, 1995); a transition at codon 71 in exon 1 (from (G) to (A) at +211 locating in the downstream region from the initial site of the transcription) that changes glycine to arginine (represented by G71R, whose type is referred to as *UGT1A1**6); a transversion at codon 229 in exon 1 that alters proline to glutamine (represented by P229Q, whose type is referred to as *UGT1A1*27*)*;* a transversion at codon 367 from (C) to (G) at position +1099 in exon 4 that converts arginine to glycine (represented by R367G, whose type is referred to as *UGT1A1**29); and a transversion (+1456, T to G) at codon 486 in exon 5 that transforms tyrosine (Y) into aspartic acid (D) (represented by Y486D, whose type is referred to as *UGT1A1*7*)*.* (Monaghan, G., Ryan, M., Seddon, R., Hume, R., and Burchell, B., Lancet, 347: 578-581, 1996, Bosma, P. J. et al., N. Engl. J. Med., 333: 1171-1175, 1995, Aono, S. et al., Lancet, 345: 958-959, 1995, Aono, S. et al., Biochem. Biophys. Res. Commun., 197: 1239-1244, 1993).

*UGT1A1*28* was distinguished from the most common allele (*UGT1A1*1*) by directly sequencing the 253-255-bps produced by PCR amplification reaction using the previously reported method (Monaghan, G. et al., Lancet, 347: 578-581, 1996, Ando, Y. et al., Pharmacogenetics, 8: 357-360, 1998).

Cycle sequencing method was performed with a dye terminator sequence reaction using an ABI PRISM 310 Genetic Analyzer (ABI Prism DNA Sequencing Kit, Perkin-Elmer, Foster City, CA).

The remaining variant sequences (such as *UGT1A1***6*) were distinguished from *UGT1A1*1* by PCR-RFLP analysis. For the analysis of exon 1, the first-step PCR amplification of a 923-bp fragment containing the exon 1 was performed in accordance with the previously reported method (Akaba, K. et al., Biochem. Mol. Biol. Int., 46: 21-26, 1998).

Subsequently, for the analysis of *UGT1A1***6* the second set of PCR amplifications was carried out using nested primers designed to amplify a 235-bp segment. The mismatched forward and reverse primers are as follows (Underlines indicate mismatched sites):
Forward primer (+178 to +210) (SEQ ID NO. 1):
   5'-CTAGCACCTGACGCCTCGTTGTACATCAGAGCC-3'
Reverse primer (+393 to +412) (SEQ ID NO. 2):
   5'-CCATGAGCTCCTTGTTGTGC-3'

The forward primer was designed to introduce a Msp I (Takara Shuzo Co., Ltd., Otsu, Japan) restriction site in *UGT1A1*1* (+209 to +212), but not in *UGT1A1*6.* The 1000-fold diluted product of the first-step PCR reaction was subjected to the 2nd step PCR using the nested PCR, wherein the sample of a a volume of 50 µl contained 0.2 mM of each deoxynucleoside triphosphate, 50 mM KCI, 10 mM Tris-HCl (pH 8.3), 1.5 mM MgCl₂, 0.5 µM of each primer, and 1.3 unit of Taq polymerase (Takara Shuzo Co., Ltd., Otsu, Japan). The PCR was conditioned as follows: 95°C for 5 min followed by 25 cycles (of 94°C for 30 s, 60°C for 40 s, and 72°C for 40 s) (PCR Thermal Cycler MP, Takara Shuzo Co., Ltd., Otsu, Japan). A 1-µl PCR amplification product was digested with 4 units of Msp I for 1 h at 37°C. DNA derived from *UGT1A1*1* was digested into 203- and 32-bp fragments, DNA from *UGT1A1*6* gave an undigested 235-bp fragment, and DNA from the heterozygous genotype gave all the three fragments.

For the sequencing of *UGT1A1***27*, another set of the second-step PCR was performed using the following hemi-nested primers designed to amplify a 399-bp segment:
Forward primer (+485 to +503) (SEQ ID NO. 3):
   5'-AGTACCTGTCTCTGCCCAC-3'
Reverse Primer (+865 to +867 and intron 1) (SEQ ID NO. 4):
   5'-GTCCCACTCCAATACACAC-3'

Two Bsr I (New England Biolabs, Inc., Beverly, MA) restriction sites exist in *UGT1A1*27* (+552 to +556 and +684 to +688), but only one site (+552 to +556) in *UGT1A1***1*. A series of PCR amplification reactions was identical with that for Msp I RFLP described above. PCR amplification products were digested with 2.5 unit of Bsr I for 1 h at 65°C. As a result, 199-, 132- and 68-bp fragments were given from *UGT1A1***27*, and or 331- and 68-bp from *UGT1A1***1*. DNA of heterozygous genotype gave all of the above four fragments.

The sequence of *UGT1A1*29* was also identified using a PCR-RFLP assay with nested primers. The first-step PCR amplification reaction encompassing exon 2, 3 and 4 was performed according to the reported method (Akaba, K. et al., Biochem. Mol. Biol. Int., 46: 21-26, 1998) with minor modifications. The forward and the reverse primers including mismatched sequences designed to amplify a 285-bp segment were used for the second-step PCR amplification reaction as follows (The underline indicates the mismatched site):
Forward primer (intron 3 and +1085 to +1098) (SEQ ID NO. 5):
   5'-TCCTCCCTATTTTGCATCTCAGGTCACCCGATGGCC-3'
Reverse primer (intron 4) (SEQ ID NO. 6):
   5'-TGAATGCCATGACCAAA-3'

The forward primer was designed to introduce a Cfr13 I (Takara Shuzo Co., Ltd., Otsu, Japan) restriction site from *UGT1A1*1* (+1095 to +1099), but not from *UGT1A1*29.* The PCR reaction reagent mixture used was the same as that used in the second-step PCR reaction for *UGT1A1*6* as described above. A PCR amplification product was digested with Cfr13 I enzyme. DNA derived from the *UGT1A1*1* was digested into 252- and 33-bp fragments, and DNA derived from *UGT1A1*29* gave an undigested 285-bp fragment.

For detection of *UGT1A1*7*, a 579-bp fragment of exon 5 was amplified by the PCR method using the primer previously reported (Akaba, K., et al., Biochem. Mol. Biol. Int., 46: 21-26, 1998).

The PCR reaction reagent mixture used was the same as that used in the second-step PCR reaction for the *UGT1A1*6.* There is a Bsr I restriction site in (+1452 to +1456) the sequence of *UGT1A1*1,* but not in *UGT1A1*7*. Therefore, by incubating with Bsr I enzyme, DNA derived from *UGT1A1*1* was digested into 365- and 214-bp fragments, and DNA derived from *UGT1A1*7* gave an undigested 579-bp fragment.

The restriction fragments resulting from the above procedures were analyzed by 4% agarose gel used electrophoresis and ethidium bromide staining. The genotyping results of every variant genotype above were confirmed by direct sequencing analyses.

The type of *UGT1A1* gene in each patient was determined in view of the results obtained.

### [Statistical Analysis]

Analysis and assessment of the correlation between severe toxicity of irinotecan and type (gene polymorphism) of *UGT1A1* gene were performed by the following statistical procedure.

For the possible factors the following are used: gender, age, performance status (PS), primary disease, presence of distant metastasis, treatment history, complications of diabetes or liver diseases, chemotherapy regimens, concurrent radiotherapy, and the intended schedule for the infusion of irinotecan and its dosage at a time. The chemotherapy regimens were categorized into 3 groups; irinotecan alone, irinotecan plus platinum (cisplatin or carboplatin), and irinotecan plus other agents (paclitaxel, docetaxel, etoposide, mitomycin C or 5-fluorouracil). The correlation (or association between potential variables was assessed using chi-square test or Fisher's exact test for categorical variables, or with Mann-Whitney U test for continuous ones. Possible variables that seemed to be associated with severe toxicity (*P* < 0.1) were to be included in the unconditional multiple logistic regression analysis. We did not include the following factors in the multivariate analysis because they highly depended on the outcome of chemotherapy: total actual dosage and use of both granulocyte colony-stimulating factor and loperamide hydrochloride. The variables in the final statistical models were chosen using forward and backward stepwise procedures at the significance level of 0.25 and 0.1, respectively. The importance of the genetic polymorphism for occurrence of severe toxicity was verified when controlling for the other variables.

We performed these analyses using JMP ver. 3.0.2 software (SAS Institute Inc., Cary, NC). A difference was considered statistically significant when the two-tailed P value was under 0.05.

### [Adverse Drug Reaction and Clinical Information]

We have reviewed the clinical information of the 118 patients. Nine (8%) and 38 (32%) patients experienced leukopenia of grade 4 (≤4.9x10⁹/liter) and grade 3 (1.9-1.0x10⁹/liter), respectively. Diarrhea was reported in 3 patients (3%) with grade 4 (hemorrhagic or dehydration) and 19 (16%) with grade 3 (watery for 5 days or more). Five of the 9 patients with grade 4 leukopenia also had grade 3/4 diarrhea, and 16 of the 22 patients with grade 3/4 diarrhea encountered grade 3/4 leukopenia. Then, we identified 26 patients who experienced severe toxicity (hereinafter referred to as "severe toxicity-experienced patients") and 92 patients who did not (hereinafter referred to as "severe toxicity-inexperienced patients") and summarized the clinical information and the severe toxicity data in Tables 2 and 3, respectively in the Drawings. Lower total amounts of actual irinotecan and more frequent use of granulocyte colony-stimulating factor or loperamide hydrochloride were observed in severe toxicity-inexperienced patients.

### [Distribution of Genotypes]

The genotypes were determined in the all 118 patients using the above methods and the result thereof is shown in Fig. 4. There was no patient having *UGT1A1*29* or *UGT1A1*7*. In addition, regarding 9 patients, the already reported result (*UGT1A1*28*) was used (Ando, Y., Saka, H., Asai, G., Sugiura, S., Simotaka, K., and Kamataki, T., Ann. Oncol., 9:845-847, 1998). In addition, regarding 117 patients, total bilirubin level before chemotherapy, and a maximum of total bilirubin level during chemotherapy term were measured, and the results thereof are also described in the table of Fig. 4.

As shown in the table in Fig. 4, the co-occurrence of the genotypic polymorphisms was found in five patients; two of them (indicated with arrow A) heterozygous for both *UGT1A1*28* and *UGT1A1*6,* and three of them heterozygous for *UGT1A1*27* and concurrently homozygous (two: indicated with arrow C) or heterozygous (one: indicated with arrow B) for *UGT1A1*28.*

The 2 patients (indicated with arrow A) heterozygous for both *UGT1A1*28* and *UGT1A1*6* had bilirubin levels within the normal range; 13.9 µmol/liter and 15.4 µmol/liter prior to therapy and 10.3 µmol/liter and 15.4 µmol/liter following the initiation of chemotherapy, respectively. Except for these 2 patients, the differences in the bilirubin levels among the genotypes were statistically significant prior to the therapy (P = 0.031, Kruskal-Wallis test) and following the initiation of therapy (P < 0.001).

The allele frequencies of *UGT1A1*28* for severe toxicity-experienced patients and severe toxicity-inexperienced patients were 0.308 (95% CI, 0.004-0.149) and 0.087 (95% CI, 0.046-0.128) respectively; and those of *UGT1A1*6*, 0.077 (95% CI, 0.004-0.149) and 0.136 (95% CI, 0.086-0.185)respectively.

The difference in allelic distribution between the patients with and without experience of severe toxicity was statistically significant for *UGT1A1*28* (*P* < 0.001) but not significant for *UGT1A1*6* (*P* > 0.2, GENEPOP ver. 3.1d software, the Laboratoire de Génétique et Environment, Montpellier, France).

### [Correlation of Genotypes and Adverse Drug Reaction]

Logistic regression analysis showed that the genotype either heterozygous or homozygous for *UGT1A1***28* proved to be a significant predictor of severe toxicity (odds ratio, 5.21; 95% CI, 1.98-13.96; P < 0.001; Table 4). Conversely, no statistical association of *UGT1A1*6* with the occurrence of severe toxicity was observed (odds ratio, 0.55; 95% CI, 0.15-1.61; P > 0.2).

Then, other factors influential in causing severe toxicity and a mutation of a genotype were compared.

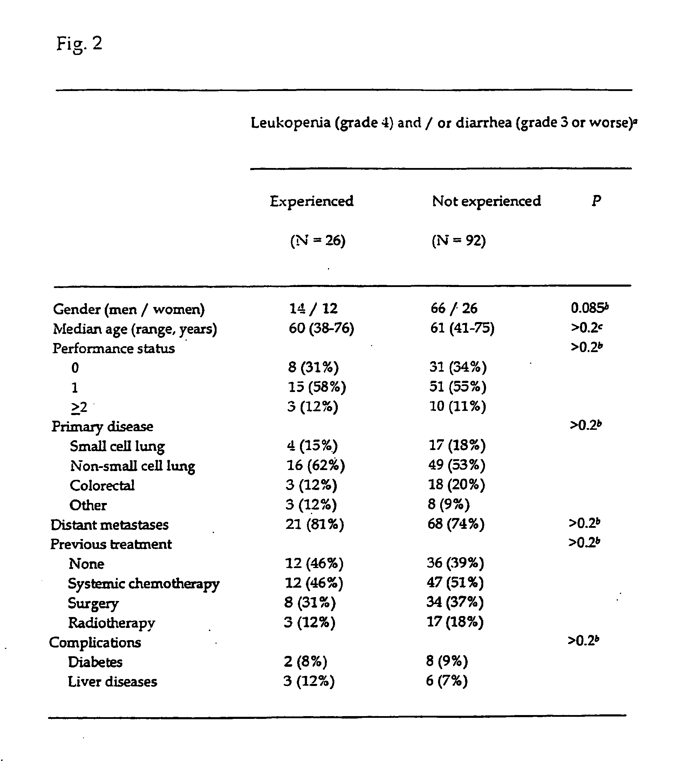

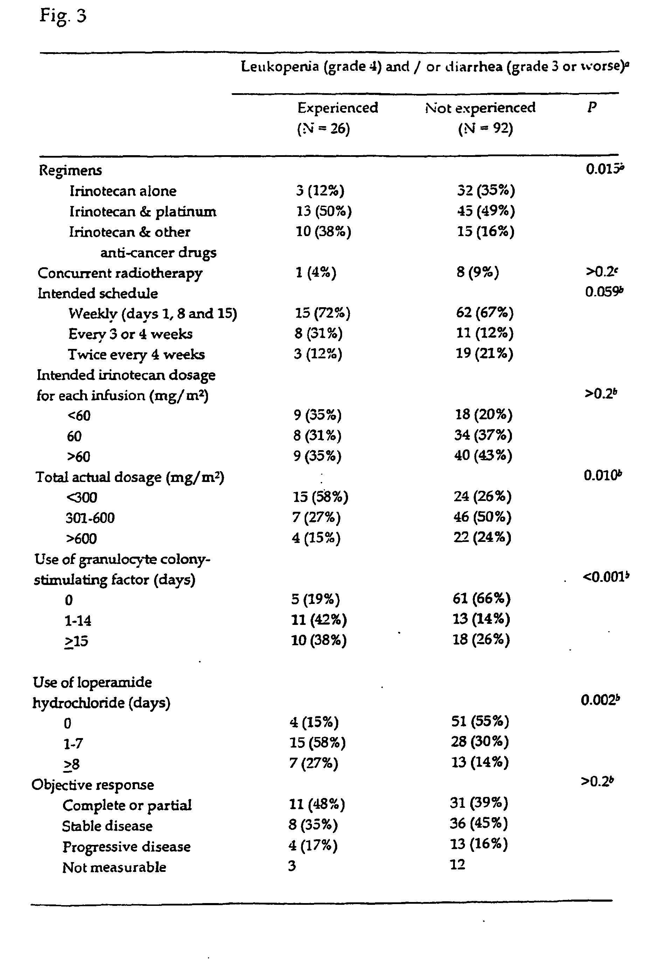

As shown in the tables in Fig. 2 and Fig. 3, the factors that seemed to affect severe toxicity adversely were "gender", "chemotherapy regimen" and "intended schedule" of irinotecan infusion. These factors were assessed for correlation or association. Significant association was found between "chemotherapy regimen" and "intended schedule" (*P* < 0.001, chi-square test), in other words, 12 of 19 patients (63%) treated with irinotecan of 3- or 4-week cycle had received additional other anticancer drugs besides irinotecan. Since "chemotherapy regimen" was the variable with stronger relationship with severe toxicity of irinotecan, we considered chemotherapy regimen" for inclusion in the statistical model.

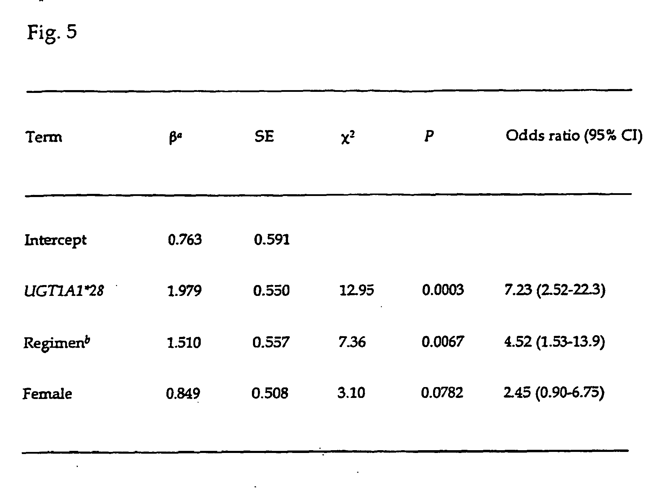

The other correlation or association seen among "chemotherapy regimen", "gender" and *"UGT1A1*28* genotype" was not significant. Being a female gender and using other anti-cancer drugs (apart from platinum) were found to be important factors of the occurrence of severe toxicity besides the *UGT1A1*28* genotype. Comparison of these two factors and *UGT1A1*28* is shown in the table of Fig. 5. As shown in the table of Fig. 5, possession of *UGT1A1*28* increases severe toxicity of irinotecan as much as 7-fold. In addition, a significant level of correlation was not recognized between being a female and severe toxicity of irinotecan. These findings clarify the clinical importance of *UGT1A1*28* as an index of UGT1A1 conjugation activity, acute exposure to irinotecan.

Among the 5 patients who had both grade 4 leukopenia and grade 3 or worse diarrhea concurrently, 2 had both *UGT1A1*28* and *UGT1A1*27,* another 2 were heterozygous for *UGT1A1*6*, and the remaining one had none of the variant genotypes analyzed (homozygous for *UGT1A1*1*). On the other hand, it is noteworthy that 4 of 5 patients (80%) who had the variant sequences both in the promoter region (*UGT1A1*28*) and in exon 1 (*UGT1A1*6* or *UGT1A1*27*) suffered from life-threatening toxicity. From the foregoing results, it is suggested that possession of *UGT1A1*28* causes severe toxicity of irinotecan at a high probability. Therefore, it can be said that a risk of expression of adverse drug reaction of irinotecan can be estimated in advance by analyzing a mutation (difference in TA repeats) in the promoter region. In addition, it is suggested that possession of both *UGT1A1*28* and either *UGT1A1*6* or *UGT1A1*27* causes severe toxicity of irinotecan at a high probability. Therefore, it can be said that a risk of expression of adverse drug reaction of irinotecan can be estimated in advance by analyzing a mutation in the promoter region and a mutation in exon 1 together.

Furthermore, the 2 patients homozygous for *UGT1A1*6* were found to be severe toxicity-inexperienced patients (the table in Fig. 4, indicated with arrow D), and all 3 patients (the same table, indicated with arrow B and C) heterozygous for *UGT1A1*27* are found to have experienced severe toxicity. From this, it is suggested that possession of *UGT1A1*27* causes severe toxicity of irinotecan at a high probability. Therefore, it can be said that a risk of expression of adverse drug reaction of irinotecan can be estimated in advance by analyzing the presence of *UGT1A1*27*, that is, polymorphism in codon 229.

The present invention is not limited to the above embodiment and Examples.

### INDUSTRIAL APPLICABILITY

According to the method of the present invention, risk of adverse drug reaction caused by the administration of a compound which is either metabolized per se by UGT1A1 enzyme or whose metabolic intermediate is metabolized by the enzyme, a representative of which is irinotecan, can be estimated in advance. As a result, it becomes possible to administer a compound (drug) in view of risks of causing adverse drug reaction every patient, and it becomes possible to reduce adverse drug reaction. In particular, a risk of the expression of adverse drug reaction such as leukopenia and diarrhea caused by the administration of irinotecan can be estimated in advance, and the risk of adverse drug reaction can be reduced.

In addition, 20% or more of the Japanese have a mutation in UGT1A1 and, for this reason, it can be considered that they may have a high risk against high toxicity of irinotecan and, therefore, it can be said that, by analyzing a genotype of *UGT1A1*, adverse drug reaction of irinotecan, in particular, in Japanese patients can be reduced.

## Claims

1. An in vitro method for estimating the risk of expression of adverse drug reaction caused by the administration of a compound which is either metabolized per se by UGT1A1 enzyme or whose metabolic intermediate is metabolized by the enzyme, which comprises a step of
(a) analyzing the number of TA repeats in the promoter region of a gene encoring UGT1A1enzyme; and
(b) analyzing the base at nucleotide position 686 of a gene encoding UGT1A1 enzyme.

2. The method of claim 1, which further comprises a step of
(c) analyzing the base at nucleotide position 211 of a gene encoding UGT1A1 enzyme.

3. The method of claim 1 or 2, wherein the step of analyzing the number of TA repeats is a step of detecting any one of 5 through 8 as the number of TA repeats.

4. The method of claim 1 or 2, wherein the step of analyzing the number of TA repeats is a step of detecting either 6 or 7 as the number of TA repeats.

5. The method of any one of claims 1 to 4, which further comprises a step of amplifying a DNA containing the TA repeating region in the promoter region of a gene encoding UGT1A1 enzyme.

6. The method of any one of claims 1 or 5, wherein the step of analyzing the base at nucleotide position 686 is a step of analyzing whether the base at nucleotide position 686 is cytosine or adenine.

7. The method of any one of claims 2 to 5, wherein the step of analyzing the base at nucleotide position 211 is a step of analyzing whether the base at nucleotide position 211 is guanine or adenine.

8. The method of any one of claims 1 to 7, which further comprises a step of amplifying a DNA containing the base at nucleotide position 686 of a gene encoding UGT1A1 enzyme.

9. The method of any one of claims 2 to 7, which further comprises a step of amplifying a DNA containing the base at nucleotide position 211 of a gene encoding UGT1A1 enzyme.

10. The method of any one of claims 1 to 9, wherein the compound is a camptothecin analogue compound.

11. The method of claim 10, wherein the camptothecin analogue compound is a camptothecin derivative.

12. The method of claim 11, wherein the camptothecin derivative is topotecan or irinotecan.

13. The method of claim 12, wherein the camptothecin derivative is irinotecan.

14. A compound as defined in any one of claims 1 or 10 to 13 for the treatment of a patient in need thereof, wherein said patient has been determined to carry a polymorphism of the UGT1A1 gene in accordance with the method of any one of claims 1 to 13.

15. Use of a first nucleic acid and a second nucleic acid in the method of any one of claims 1 to 14, wherein
(a) said first nucleic acid is used for analyzing the number of TA repeats in the promoter region of a gene encoding UGT1A1 enzyme, wherein said first nucleic acid hybridizes specifically with a DNA fragment derived from the region which contains the bases of the TA repeating region of a gene encoding UGT1A1 enzyme and wherein said DNA fragment can be amplified by PCR method using
(i) the primers of SEQ ID No. 7 and SEQ ID No. 8;
(ii) the primers of SEQ ID No. 9 and SEQ ID No. 10; and/or
(iii) the primers of SEQ ID No. 1 and SEQ ID No. 2; and
(b) said second nucleic acid is used for analyzing the base at nucleotide position 686 of a gene encoding UGT1A1 enzyme, wherein said second nucleic acid hybridizes specifically with a DNA fragment derived from the region which contains the base at nucleotide position 686 of a gene encoding UGT1A1 enzyme and wherein said DNA fragment can be amplified by PCR method using the primers of SEQ ID No. 3 and SEQ ID No. 4.

16. Use of a kit in the method of any one of claims 1 to 14, said kit comprising
(a) a nucleic acid for analyzing the number of TA repeats in the promoter region of a gene encoding UGT1A1 enzyme; and
(b) a nucleic acid for analyzing the base at nucleotide position 686 of a gene encoding UGT1A1 enzyme.

17. The use of claim 16, wherein said kit further comprises
(c) a nucleic acid for analyzing the base at nucleotide position 211 of a gene encoding UGT1A1 enzyme.

18. The use of claim 16 or 17, wherein the compound is a camptothecin analogue compound.

19. The use of claim 18, wherein the camptothecin analogue compound is a camptothecin derivative.

20. The use of claim 19, wherein the camptothecin derivative is either topotecan or irinotecan.

21. The use of claim 20, wherein the camptothecin derivative is irinotecan.

22. The use of claim 16, wherein said kit further comprises a reagent for amplifying a DNA containing the base at nucleotide position 686 of a gene encoding UGTIA1 enzyme.

23. The use of claim 22, wherein said kit further comprises a reagent for amplifying a DNA containing a TA repeating region in the promoter region of a gene encoding UGT1A1 enzyme and/or a nucleic acid for analyzing the base at nucleotide position 211 of a gene encoding UGT1A1 enzyme and a reagent for amplifying a DNA containing the base at nucleotide position 211 of a gene encoding UGT1A1 enzyme.

## Patentansprüche

1. Ein *in vitro*-Verfahren zum Einschätzen des Risikos des Auftretens einer unerwünschten Arzneimittelreaktion, verursacht durch die Verabreichung einer Verbindung, die entweder per se durch das Enzym UGT1A1 metabolisiert wird oder deren metabolisches Zwischenprodukt durch das Enzym metabolisiert wird, wobei das Verfahren einen Schritt
(a) des Analysierens der Anzahl von TA-Wiederholungen in der Promotorregion eines das Enzym UGT1A1 kodierenden Gens; und
(b) des Analysierens der Base an Nukleotidposition 686 eines das Enzym UGT1A1 kodierenden Gens
umfasst.

2. Verfahren nach Anspruch 1, des Weiteren umfassend einen Schritt
(c) des Analysierens der Base an Nukleotidposition 211 eines das Enzym UGT1A1 kodierenden Gens.

3. Verfahren nach den Ansprüchen 1 oder 2, wobei der Schritt des Analysierens der Anzahl an TA-Wiederholungen ein Schritt zum Detektieren von 5 bis einschließlich 8 TA-Wiederholungen ist.

4. Verfahren nach den Ansprüchen 1 oder 2, wobei der Schritt des Analysierens der Anzahl der TA-Wiederholungen ein Schritt zum Detektieren von 6 oder 7 TA-Wiederholungen ist.

5. Verfahren nach einem der Ansprüche 1 bis 4, des Weiteren umfassend einen Schritt zum Amplifizieren einer DNA, welche die Region der TA-Wiederholungen in der Promotorregion eines das Enzym UGT1A1 kodierenden Gens enthält.

6. Verfahren nach einem der Ansprüche 1 oder 5, wobei der Schritt des Analysierens der Base an der Nukleotidposition 686 ein Schritt zum Analysieren ist, ob die Base an Nukleotidposition 686 Cytosin oder Adenin ist.

7. Verfahren nach einem der Ansprüche 2 bis 5, wobei der Schritt zum Analysieren der Base an Nukleotidposition 211 ein Schritt zum Analysieren ist, ob die Base an Nukleotidposition 211 Guanin oder Adenin ist.

8. Verfahren nach einem der Ansprüche 1 bis 7, des Weiteren umfassend einen Schritt zum Amplifizieren einer die Base an Nukleotidposition 686 enthaltenden DNA eines Gens, welches das Enzym UGT1A1 kodiert.

9. Verfahren nach einem der Ansprüche 2 bis 7, des Weiteren umfassend einen Schritt zum Amplifizieren einer die Base an Nukleotidposition 211 enthaltenden DNA eines Gens, welches das Enzym UGT1A1 kodiert

10. Verfahren nach einem der Ansprüche 1 bis 9, wobei die Verbindung eine Camptothecinanalogverbindung ist.

11. Verfahren nach Anspruch 10, wobei die Camptothecinanalogverbindung ein Camptothecinderivat ist.

12. Verfahren nach Anspruch 11, wobei das Camptothecinderivat entweder Topotecan oder Irinotecan ist.

13. Verfahren nach Anspruch 12, wobei das Camptothecinderivat Irinotecan ist.

14. Eine Verbindung wie in einem der Ansprüche 1 oder 10 bis 13 definiert zur Verwendung bei der Behandlung eines einer solchen Behandlung bedürfenden Patienten, wobei bei dem Patienten festgestellt wurde, dass er einen Polymorphismus des UGT1A1-Gens trägt, in Übereinstimmung mit dem Verfahren nach einem der Ansprüche 1 bis 13.

15. Verwendung einer ersten Nukleinsäure und einer zweiten Nukleinsäure im Verfahren nach einem der Ansprüche 1 bis 14, wobei
(a) die erste Nukleinsäure dazu verwendet wird, die Anzahl der TA-Wiederholungen in der Promotorregion eines das Enzym UGT1A1 kodierenden Gens zu analysieren, wobei die erste Nukleinsäure spezifisch mit einem DNA-Fragment hybridisiert, das aus der Region stammt, welche die Basen der Region der TA-Wiederholungen eines das Enzym UGT1A1 kodierenden Gens enthält, und wobei das DNA-Fragment mittels eines PCR-Verfahrens amplifiziert werden kann, unter Verwendung
(i) der Primer mit SEQ ID NR: 7 und SEQ ID NR: 8;
(ii) der Primer mit SEQ ID NR: 9 und SEQ ID NR: 10; und/oder
(iii) der Primer mit SEQ ID NR: 1 und SEQ ID NR: 2; und
(b) die zweite Nukleinsäure dazu verwendet wird, die Base an Nukleotidposition 686 eines das Enzym UGT1A1 kodierenden Gens zu analysieren, wobei die zweite Nukleinsäure spezifisch mit einem DNA-Fragment hybridisiert, das aus der Region stammt, welche die Base an Nukleotidposition 686 eines das Enzym UGT1A1 kodierenden Gens enthält, und wobei das DNA-Fragment mittels eines PCR-Verfahrens unter Verwendung der Primer mit SEQ ID NR: 3 und SEQ ID NR: 4 amplifiziert werden kann.

16. Verwendung eines Kits in dem Verfahren nach einem der Ansprüche 1 bis 14, wobei das Kit
(a) eine Nukleinsäure zum Analysieren der Anzahl der TA-Wiederholungen in der Promotorregion eines das Enzym UGT1A1 kodierenden Gens; und
(b) eine Nukleinsäure zum Analysieren der Base an Nukleotidposition 686 eines das Enzym UGT1A1 kodierenden Gens
umfasst.

17. Verwendung nach Anspruch 16, wobei das Kit des Weiteren
(c) eine Nukleinsäure zum Analysieren der Base an Nukleotidposition 211 eines das Enzym UGT1A1 kodierenden Gens
umfasst.

18. Verwendung nach Anspruch 16 oder 17, wobei die Verbindung eine Camptothecinanalogverbindung ist.

19. Verwendung nach Anspruch 18, wobei die Camptothecinanalogverbindung ein Camptothecinderivat ist.

20. Verwendung nach Anspruch 19, wobei das Camptothecinderivat entweder Topotecan oder Irinotecan ist.

21. Verwendung nach Anspruch 20, wobei das Camptothecinderivat Irinotecan ist.

22. Verwendung nach Anspruch 16, wobei das Kit des Weiteren ein Reagens zum Amplifizieren einer DNA umfasst, welche die Base an Nukleotidposition 686 eines das Enzym UGT1A1 kodierenden Gens enthält.

23. Verwendung nach Anspruch 22, wobei das Kit des Weiteren umfasst: ein Reagens zum Amplifizieren einer DNA, welche eine Region von TA-Wiederholungen in der Promotorregion eines das Enzym UGT1A1 kodierenden Gens enthält und/oder eine Nukleinsäure zum Analysieren der Base an Nukleotidposition 211 eines das Enzym UGT1A1 kodierenden Gens und ein Reagens zum Amplifizieren einer DNA, welche die Base an Nukleotidposition 211 eines das Enzym UGT1A1 kodierenden Gens enthält.

## Revendications

1. Procédé in vitro permettant d'estimer le risque d'expression d'une réaction médicamenteuse indésirable provoquée par l'administration d'un composé, soit qui est métabolisé lui-même par l'enzyme UGT1A1, soit dont un intermédiaire métabolique est métabolisé par l'enzyme, qui comprend une étape consistant à :
(a) analyser le nombre de séquences TA répétées dans la région promotrice d'un gène codant pour l'enzyme UGT1A1 ; et
(b) analyser la base qui se trouve à la position nucléotidique 686 d'un gène codant pour l'enzyme UGT1A1.

2. Procédé selon la revendication 1, qui comprend en outre une étape consistant à :
(c) analyser la base qui se trouve à la position nucléotidique 211 d'un gène codant pour l'enzyme UGT1A1.

3. Procédé selon les revendications 1 ou 2, dans lequel l'étape d'analyse du nombre de séquences TA répétées est une étape de détection de tout nombre compris entre 5 et 8 en tant que nombre de séquences TA répétées.

4. Procédé selon les revendications 1 ou 2, dans lequel l'étape d'analyse du nombre de séquences TA répétées est une étape de détection du nombre 6 ou 7 en tant que nombre de séquences TA répétées.

5. Procédé selon l'une quelconque des revendications 1 à 4, qui comprend en outre une étape consistant à amplifier un ADN contenant la région de répétition de TA dans la région promotrice d'un gène codant pour l'enzyme UGT1A1.

6. Procédé selon l'une quelconque des revendications 1 à 5, dans lequel l'étape d'analyse de la base qui se trouve à la position nucléotidique 686 est une étape consistant à analyser si la base qui se trouve à la position nucléotidique 686 est une cytosine ou une adénine.

7. Procédé selon l'une quelconque des revendications 2 à 5, dans lequel l'étape d'analyse de la base qui se trouve à la position nucléotidique 211 est une étape consistant à analyser si la base qui se trouve à la position nucléotidique 211 est une guanine ou une adénine.

8. Procédé selon l'une quelconque des revendications 1 à 7, qui comprend en outre une étape consistant à amplifier un ADN contenant la base qui se trouve à la position nucléotidique 686 d'un gène codant pour l'enzyme UGT1A1.

9. Procédé selon l'une quelconque des revendications 2 à 7, qui comprend en outre une étape consistant à amplifier un ADN contenant la base qui se trouve à la position nucléotidique 211 d'un gène codant pour l'enzyme UGT1A1.

10. Procédé selon l'une quelconque des revendications 1 à 9, dans lequel le composé est un analogue de la camptothécine.

11. Procédé selon la revendication 10, dans lequel l'analogue de la camptothécine est un dérivé de la camptothécine.

12. Procédé selon la revendication 11, dans lequel le dérivé de la camptothécine est le topotécan ou l'irinotécan.

13. Procédé selon la revendication 12, dans lequel le dérivé de la camptothécine est l'irinotécan.

14. Composé tel que défini dans l'une quelconque des revendications 1 ou 10 à 13, destiné à être utilisé dans le traitement d'un patient en ayant besoin, ledit patient ayant été identifié comme porteur d'un polymorphisme du gène UGT1A1 conformément au procédé selon l'une quelconque des revendications 1 à 13.

15. Utilisation d'un premier acide nucléique et d'un second acide nucléique dans le procédé selon l'une quelconque des revendications 1 à 14, dans laquelle :
(a) ledit premier acide nucléique est utilisé pour analyser le nombre de séquences TA répétées dans la région promotrice d'un gène codant pour l'enzyme UGT1A1, ledit premier acide nucléique s'hybridant spécifiquement à un fragment d'ADN dérivé de la région qui contient les bases de la région de répétition de TA d'un gène codant pour l'enzyme UGT1A1 et ledit fragment d'ADN pouvant être amplifié par la méthode PCR en utilisant :
(i) les amorces de SEQ ID N° 7 et SEQ ID N° 8 ;
(ii) les amorces de SEQ ID N° 9 et SEQ ID N° 10 ; et/ou
(iii) les amorces de SEQ ID N° 1 et SEQ ID N° 2 ; et
(b) ledit second acide nucléique est utilisé pour analyser la base qui se trouve à la position nucléotidique 686 d'un gène codant pour l'enzyme UGT1A1, ledit second acide nucléique s'hybridant spécifiquement à un fragment d'ADN dérivé de la région qui contient la base qui se trouve à la position nucléotidique 686 d'un gène codant pour l'enzyme UGT1A1 et ledit fragment d'ADN pouvant être amplifié par la méthode PCR en utilisant les amorces de SEQ ID N° 3 et SEQ ID N° 4.

16. Utilisation d'une trousse dans le procédé selon l'une quelconque des revendications 1 à 14, ladite trousse comprenant :
(a) un acide nucléique permettant l'analyse du nombre de séquences TA répétées dans la région promotrice d'un gène codant pour l'enzyme UGT1A1 ; et
(b) un acide nucléique permettant l'analyse de la base qui se trouve à la position nucléotidique 686 d'un gène codant pour l'enzyme UGT1A1.

17. Utilisation selon la revendication 16, dans laquelle ladite trousse comprend :
(c) un acide nucléique permettant l'analyse de la base qui se trouve à la position nucléotidique 211 d'un gène codant pour l'enzyme UGT 1 A 1.

18. Utilisation selon la revendication 16 ou 17, dans laquelle le composé est un analogue de la camptothécine.

19. Utilisation selon la revendication 18, dans laquelle l'analogue de la camptothécine est un dérivé de la camptothécine.

20. Utilisation selon la revendication 19, dans laquelle le dérivé de la camptothécine est le topotécan ou l'irinotécan.

21. Utilisation selon la revendication 20, dans laquelle le dérivé de la camptothécine est l'irinotécan.

22. Utilisation selon la revendication 16, dans laquelle ladite trousse comprend en outre un réactif permettant l'amplification d'un ADN contenant la base qui se trouve à la position nucléotidique 686 d'un gène codant pour l'enzyme UGT1A1.

23. Utilisation selon la revendication 22, dans laquelle ladite trousse comprend en outre un réactif permettant l'amplification d'un ADN contenant une région de répétition de TA dans la région promotrice d'un gène codant pour l'enzyme UGT1A1 et/ou un acide nucléique permettant l'analyse de la base qui se trouve à la position nucléotidique 211 d'un gène codant pour l'enzyme UGT1A1 et un réactif permettant l'amplification d'un ADN contenant la base qui se trouve à la position nucléotidique 211 d'un gène codant pour l'enzyme UGT1A1.
